# EUROPEAN PATENT APPLICATION

(11) **EP 4 039 291 A1**
(43) Date of publication of application: **10.08.2022**
(21) Application number: 22155274.8
(22) Date of filing: 04.02.2022
(51) Int. Cl.: A61M 5/14, A61M 5/168, A61M 5/36, A61M 39/08, A61M 5/142

(54) **CONNECTED INFUSION PUMP DEVICE**

(30) Priority: 04.02.2021 EP 21020053
(71) Applicant: Micrel Medical Devices S.A., Konstantinoupoleos 42 Karela Industrial Area 19441 Koropi (GR)
(72) Inventor: TSOUKALIS, Achilleas, Papagou 15669 Attiki (GR)
(74) Representative: Eisenführ Speiser

(57) **Abstract**

An infusion pump device comprises a pump unit (30, 40) having an inlet (32) and an outlet (33), a connector being in fluid communication with the inlet (32) of said pump unit (30, 40), a medication reservoir accommodation portion (4) for accommodating a medication reservoir (14) having a label or tag (16) so that an outlet (18) of the medication reservoir (14) is to be coupled to said connector, and a reader (5) which is provided at said medication reservoir accommodation portion (4) so that the reader (5) is adapted to read a label or tag (16) of the medication reservoir (14) when arranged at said medication reservoir accommodation portion (4).

## Description

Ambulatory infusion pumps are used outside and inside the hospital, for smaller volume drug delivery like 100 ml morphine to palliative care patients, or higher volume drug delivery like in case of parenteral nutrition where large volumetric pumps (LVP) are too big to carry. A big use of ambulatory infusion pumps is with chronic patients at home or with therapies that last long with the patient at home.

LVP pumps, the standard infusion pumps for hospital use, have since decades a safety feature named drug library where the drugs used in a care area of the hospital are stored in a database with soft and hard infusion rate limits depending on the combination "therapy + patient type or therapy subtype (therapy specificity) + drug used", to prevent erroneous programming e.g. by a nurse with fatal results.

Soft limits in general are overridden with a warning, whereas hard limits cannot be.

These databases are made for hospital use where many different therapies are treated by a pump even within a week time. Drug libraries are not adapted for ambulatory infusion pumps that are mostly used at home by chronic patients. It is the aim of the present invention to provide easier means for infusion pump use with safety at home.

In hospitals a nurse is watching the patient's health, and complications are quickly resolved, while this is not the case with home care. It is the aim of the present invention to provide means for tele-monitoring therapy feedback from home therapies.

In hospitals nurses are trained, and there is a organizational structure to watch and guide their work, while in home care nurses are free lancers with no superiors so that errors are more frequent. It is the aim of the present invention to provide means to keep errors minimal at home care use of ambulatory infusion pumps.

### BRIEF DESCRIPTION OF THE INVENTION

In order to achieve the aforementioned and further objects, according to a first aspect of the present invention, there is provided an infusion pump device, comprising a pump unit having an inlet and an outlet, a connector being in fluid communication with the inlet of said pump unit, a medication reservoir accommodation portion for accommodating a medication reservoir having a label or tag so that an outlet of the medication reservoir is to be coupled to said connector, and a reader which is provided at said medication reservoir accommodation portion so that the reader is adapted to read a label or tag of the medication reservoir when arranged at said medication reservoir accommodation portion.

Preferred embodiments and modifications of the first aspect of the present invention are defined in the dependent claims 2 to 23.

In the meaning of the present invention, the "pump unit" is defined as comprising a fluidic line or passage part for guiding a medication fluid or drug from the inlet to the outlet of the pump unit and a mechanism for pumping the medication or drug through the fluidic line or passage part, wherein the fluidic line or passage part preferably comprises a disposable infusion set with a resilient tubing.

Preferably, the infusion pump device further comprises a housing which includes at least said pump unit, said medication reservoir accommodation portion and said reader.

According to a further preferred embodiment of the first aspect, said reader is further adapted to read the tag or label of a patient as long as a medication reservoir is absent from said medication reservoir accommodation portion.

According to a further preferred embodiment of the first aspect, said medication reservoir accommodation portion comprises a compartment adapted to accommodate a medication reservoir.

According to a modification of the above embodiment, said compartment comprises a bottom and sidewalls, and wherein said reader is provided at said bottom and/or at at least one of said sidewalls.

According to a further preferred embodiment of the first aspect, said reader is an RFID/NFC and/or barcode reader.

According to a further preferred embodiment of the first aspect, the infusion pump device further comprises a communication unit, in particular a wireless communication unit (like a WiFi and/or a GSM/GPS/3G/4G/5G etc. cellular wireless communication unit), adapted to communicate with a remote server.

According to a further preferred embodiment of the first aspect, the infusion pump device comprises a storing unit adapted to store at least a drug library downloaded from the remote server and including a list of different drugs, and an infusion protocol list downloaded from the remote server and including different specific infusion protocols each of which requires at least one specific drug taken from said drug library and defines a specific drug delivery and/or application to a patient.

According to a further preferred embodiment of the first aspect, the infusion pump device comprises an input unit adapted to enable a selection of a predetermined specific infusion protocol from the infusion protocol list.

According to a further preferred embodiment of the first aspect, said input unit is further adapted to enable a modification of the predetermined specific infusion protocol, said storing unit is further adapted on the basis of said modification to amend said predetermined specific infusion protocol to a modified specific infusion protocol in the infusion protocol list stored, and said input unit is further adapted to then enable a selection of a predetermined specific infusion protocol being said modified specific infusion protocol from the infusion protocol list. So, once a protocol is modified, this modification is stored, and next time when the protocol list is provided or shown, the modified protocol will be output to be used for starting the pumping mechanism.

According to a further preferred embodiment of the first aspect, the infusion pump device further comprises a processing unit adapted to check if the read drug data read by said reader and indicating the characteristics of the drug included in the medication reservoir matches with the requirements of the predetermined specific infusion protocol and only in case there is a match allows said pumping mechanism to be started. For example, "drug morphine concentration 2 mg/ml" is read by the reader from the tag or label of the drug bag or medication reservoir, "max dosage 0.2 mg/kg every 4 hours" is read from the drug library, the patient data weight of 80 kg is read, and a suggested infusion protocol "2 ml/h" is read so that as a result a match is found correct as maximum volume for weight 0.2 * 80 = 16 mg at 4 hours = 4 mg/h = 2 ml/h for a concentration of 2 mg/ml.

According to a further preferred embodiment of the first aspect, the infusion pump device further comprises a user interface, wherein said processing unit is adapted on the basis of the read drug data to find only those specific infusion protocols which are relevant for the read drug and to present them by said user interface. In particular, in the drug library a selection is made for each therapy where a drug verification is needed so that the processing unit needs to read the drug label and to check it before an infusion protocol is allowed to be infused by starting said pump unit.

According to a further preferred embodiment of the first aspect, said processing unit is further adapted to reveal from said relevant specific infusion protocols the specific infusion protocol which has been most used within a given time interval in the past and to present this specific infusion protocol at a first place in an order by said user interface. So, after reading, the most used infusion protocols for the drug just read are appearing first in the selected or allowed protocol list, i.e. drugs with the same name and concentration with regard to the read drug, and the protocol list is again reduced as usual in the first place (before reading) with the given therapy and profile.

According to a further preferred embodiment of the first aspect, said processing unit is further adapted to select only those specific infusion protocols which in particular require the administration of a drug with the same name and concentration as with the read drug.

According to a further preferred embodiment of the first aspect, said storing unit is further adapted to store a patient list downloaded from the remote server, and said input unit is further adapted to enable a selection of a specific patient profile from the patient list.

According to a further preferred embodiment of the first aspect, said processing unit is further adapted to provide for said selected specific patient profile a patient-medication link for downloading a relative infusion protocol and a 5R list including patent verification, drug, infusion protocol, time of the start of the infusion and delivery route from the remote server or for reading them from the label or tag of the medication reservoir, and to validate the specific patient and drug. The 5R list is mainly used in a hospital and is difficult to be implemented without automatic tools such as a reader. By means of automatic tools the patient and the drug tag or label can be scanned, and then the provided infusion protocol, the delivery route and the time to start the infusion are just to be validated, so that the process is simplified and errors are avoided. The 5R list is downloaded from the remote server, usually provided for a CERNER/EPIC type of therapy calendars and then transferred to an app (like the "Micrel Care" app).

According to a further preferred embodiment of the first aspect, said processing unit is further adapted, after having read the drug data by said reader, to find a predetermined specific infusion protocol from the infusion protocol list by using the specific patient profile, and in case said patient profile indicates a chronic patient to omit this step if the predetermined specific infusion protocol has already used once and the patient is the same.

According to a further preferred embodiment of the first aspect, the drug library enables the correct finding of an infusion protocol by using a three step process wherein a therapy is selected from a list, then a profile is selected from another list, and then this combination reduces the list of relevant drugs to a few ones while the total list is rather big and difficult to search through. Whereas in a hospital usually the pumps are used for several therapies changing from patient to patient, in homecare a pump can be used several times or all the time for one therapy and a few drugs related to the therapy and user profile. While a protocol list has therapy and profile on the protocol list name and drug on the protocol name, after the download of a list for the specific therapy and profile it has just to be checked a drug from the protocol list to be infused. So, there are less steps in the user interface to be carried out so as to find a correct infusion protocol, resulting in easing the use for chronic patients since it is dealt with a few protocols only and where therapy and profile are not changed by rendering it a mono-therapy pump for a dedicated user. For example, a chronic patient for parenteral nutrition therapy usually has a pump exclusively for himself and his daily therapy. Whereas a conventional drug library does not make the use of the pump easy, according to the preferred embodiment a dedicated short protocol list is provided which can include 3 or 4 protocols like total parenteral nutrition (TPN), lipids infusion, amino acids infusion, and hydration. Once an infusion protocol such as infusion rate is changed, it reappears next time for the same infusion protocol as the user and his condition are the same, so that it is not needed to reprogram the pump each time since a selection from a short protocol list is an easier process than a normal pump programming. In other words, once a therapy is selected, the database reduces the drug list, and once a user profile is selected, the list is further reduced making easier a drug to be found. So, the therapy and user profile are already in place before reading, and it is not needed all time to enter the therapy and user profile for a chronic patient who is subject to a monotherapy wherein there is always the same profile.

According to a further preferred embodiment of the first aspect, said processing unit is further adapted to validate the read drug by using a 5R list including patent verification, drug, infusion protocol, time of the start of the infusion and delivery route downloaded from the remote server.

According to a further preferred embodiment of the first aspect, said processing unit is further adapted to detect from the read drug data the content of the drug as a characteristic and preferably in addition patient specific and/or infusion specific data and/or a link for downloading a 5R list including patent verification, drug, infusion protocol, time of the start of the infusion and delivery route from the remote server.

According to a further preferred embodiment of the first aspect, said processing unit is further adapted on the basis of a selection of the therapy and the drug as well as a profile defining a subcategory of the selected therapy to provide a selection of predetermined specific infusion protocols to be used. Therapy is usually a large item, so that a profile is provided which defines a subcategory of that therapy where infusion protocols and even drugs to be used are different. The subcategory can be patient dependent as adult/child or gender or weight or a subcategory of the therapy itself, as e.g. several ones exist for regional analgesia. All these items are arbitrary for the design of the drug library at the remote server. They are not done at pump level just used with the pump so as to easier find a short list of infusion protocols to be chosen.

According to a further preferred embodiment of the first aspect, the infusion pump device comprises a location detection unit adapted to receive and process the signals from said communication unit and on the basis of said signals to determine the actual location of the infusion pump device.

According to a preferred modification of the above embodiment, said location detection unit is further adapted to store the determined actual location.

According to a still further preferred modification of the above embodiment, the infusion pump device further comprises a display, wherein said location detection unit is further adapted to still operate even when said display is deactivated. According to a second aspect of the present invention, there is provided a medication reservoir for the infusion pump device according to the first aspect, comprising a label or tag and including a drug pre-filled in a pharmaceutical company or compounded in a hospital which drug in particular comprises an analgesic medication.

So, according to a preferred embodiment, the pump comprises a battery compartment with a disposable or rechargeable main battery and rechargeable secondary batteries so that the pump can even run during battery change, an electronic control with LCD display and sensors to help verify infusion conditions like upstream and downstream pressure, air in line, motor speed, voltages etc. The pump further comprises an integrated lockable drug bag compartment in different sizes for dedicated smaller and larger infusion bag sets and an RFID reader so that any tagged bag that is put into the compartment can be read so as to render an infusion protocol selection automated and to exclude medication errors. Further provided is a WiFi and GSM communication with an external cloud based server to transmit infusion data, therapy feedback data and alarms and to receive drug library and protocol list data through a secure encrypted communication and to perform location tracking for a lost pump or other devices in hospitals. Location tracking associated with an RFID recognition of a drug and infusion data complete a compliance file to be sent back to the patient's electronic health record.

A drug library is a list of drugs per concentration and their soft and hard infusion limits for a therapy, specialized for a specific patient or delivery route or other therapy specificity or variant called therapy profile. Therefore, limits for a child and an adult are different for the same drug and therapy. Since the drug library design is difficult to be implemented in a pump with limited keys, it is set up on a PC and transferred to the pump by cable or wirelessly. According to a preferred embodiment of the present invention, the PC is a terminal of a cloud-based monitoring and programming application (with commercial name "MicrelCare"). There are different drug libraries (limits and drugs used) per hospital care area or home care facility, each having preferences on safety limits.

According to a preferred embodiment of the present invention, while the standard infusion protocol search path "therapy + profile (subcategory of therapy with specificities for a patient or practice) + drug" is used, there are added features being especially useful at home care setting. Drug libraries, which have been first invented for bedside (LVP) pumps in a hospital multitherapy environment, are now needed to be adapted for ambulatory pumps and easiest use with home therapies.

So, the therapist can select some protocols from the drug library and put them in a most used protocol list as a separate menu. In this way, a chronic patient who takes a parenteral nutrition at home does not need each time to carry out a full library search, but only needs to select a hydration or yellow or white bag drug protocol for his next infusion. Since the drug library is for hospital use, each change does not overwrite the protocol in the drug library. In contrast thereto, according to the present invention, the protocol list is subject to such an overwriting so that each time a patient or user modifies a protocol from a protocol list which appears next time and is then used as default.

So far, drug libraries include a pump configuration, i.e. general pump settings, for infusion at protocol level. In contrast thereto, according to a preferred embodiment of the present invention, the drug libraries include the pump configuration for infusion at therapy level resulting in easing home care use that usually is for one therapy only.

According to a further embodiment of the present invention, with the drug library setting there are standard questions for the user as therapy feedback to be chosen by the doctor for a specific therapy profile which can be more standard or may be a therapy combination treatment and needs customization. The frequency and time of the appearance of the questions on the pump screen is a definable parameter in the drug library. Since the pump is interconnected as an internet of things (loT) device, the answers together with infusion data and alarms are written in a database of a remote server and presented per infusion or series of consequent infusions, so that the doctor can better visualize the therapy progress since the feedback is presented as a graph, too. In case of e.g. a parenteral nutrition, the doctor can see which nutrition types have been taken last time, e.g. last month, with downstream pressure and catheter near occlusion alarm algorithm results (e.g. on the basis of pressure data processing to predict imminent occlusion), patient weight evolution, dry feeling, urine output, or glucose level side effects in case of an erroneous ramp type infusion setting. A new protocol can be sent via the internet, and then the pump reads it and sends it back to the server so that the doctor is assured that the correct protocol has been arrived and stored on the pump. Then he sends an acknowledgement so that the user can use it safely. This way a total therapy feedback and treatment correction is made remotely.

According to a further preferred embodiment of the present invention, the pump also comprises an RFID reader, which is close to the drug bag compartment and reads an RFID tag embedded or adhesively put to the bag, and the pump automatically selects from the drug library the correct protocol for the user that can also be scanned by the RFID reader by using a patient tag. This embodiment is intended to be used for prefilled drug bags (configured and sold by pharmaceutical companies) wherein the RFID tag is provided on the drug bag surface, or for drug bags including compounded drugs prepared in a compounder facility or hospital pharmacy wherein said bags comprise a self-adhesive label including written information combined with RFID data. In the database there is created a No. to be read from an RFID tag indicating a customized (compounded) drug that is not present in general barcode systems. Whereas in several countries two nurses are needed to make sure that the correct drug and protocol are delivered to a patient, according to a preferred embodiment of the present invention, the RFID reader provides a verification ID back to the server so that the system needs to allow only a single nurse for a treatment using telemedicine. Errors in morphine preparation onsite due to both dilution errors and programming erroneous concentration are frequent and sometimes fatal. The present invention prevents erroneous drug delivery while easing the use.

### BRIEF DESCRIPTION OF THE DRAWINGS

- Fig. 1: shows a perspective top view (a) and a perspective side view (b) of a pump according to a preferred embodiment.
- Fig. 2: shows the pump of Fig. 1 with its drug bag compartment being opened.
- Fig. 3: shows a portion of the pump of Fig. 1 with its battery and GSM SIM card compartment.
- Fig. 4: shows an embodiment of a drug bag with an RFID tag prefilled by a pharmaceutical company and including e.g. analgesics.
- Fig. 5: shows a schematic block diagram of an internal electronic circuit of the pump of Fig. 1 with the most relevant components.
- Fig. 6: shows a therapies menu window further indicating an input field for adding a therapy.
- Fig. 7: shows a drugs menu window.
- Fig. 8: shows a drug library menu window indicating general therapy settings.
- Fig. 9: shows the drug library menu window with an input field for editing delivery profiles and drugs per therapy.
- Fig. 10: shows the drug library menu window with an input field for editing protocol and limits for a chosen therapy-profile-drug.
- Fig. 11: shows a protocol lists menu window with an input field for adding a protocol list.
- Fig. 12: shows a protocol lists menu window indicating a general therapy settings.
- Fig.13: shows a protocol lists menu window with an input field for editing delivery infusion parameters and limits.
- Fig. 14: shows a questionnaires menu window giving several questionnaire types per therapy.
- Fig.15: shows an input window for adding questions.
- Fig.16: shows a pump files menu window giving files to be uploaded to pumps per therapy.
- Fig.17: shows the pump files menu window indicating a review and release information about the file (quality management).

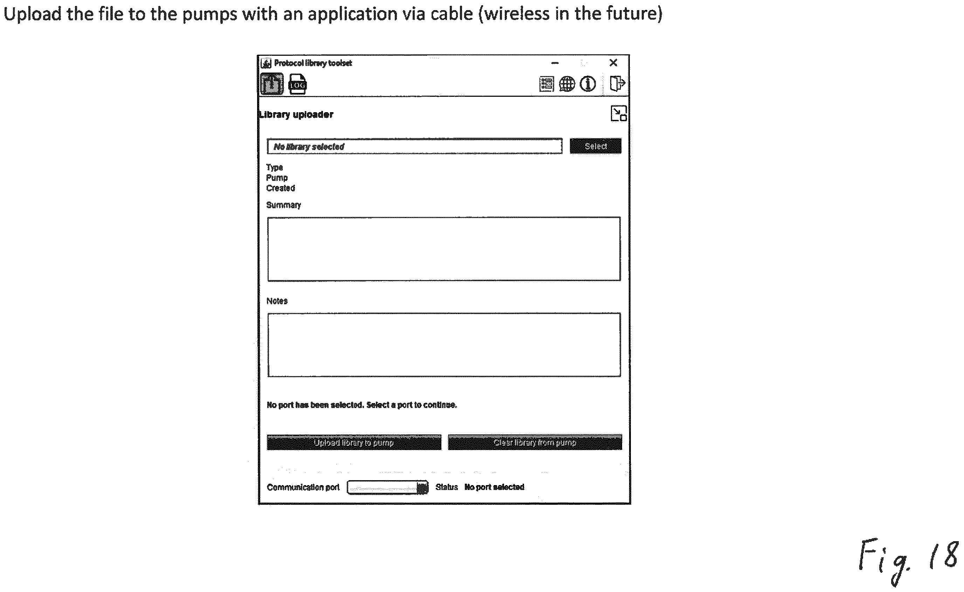

- Fig.18: shows a window indicating the upload of a file to the pump.
- Fig. 19: shows an example of a display indicating a drug library and a protocol list on the pump.

### DESCRIPTION OF A PREFERRED EMBODIMENT

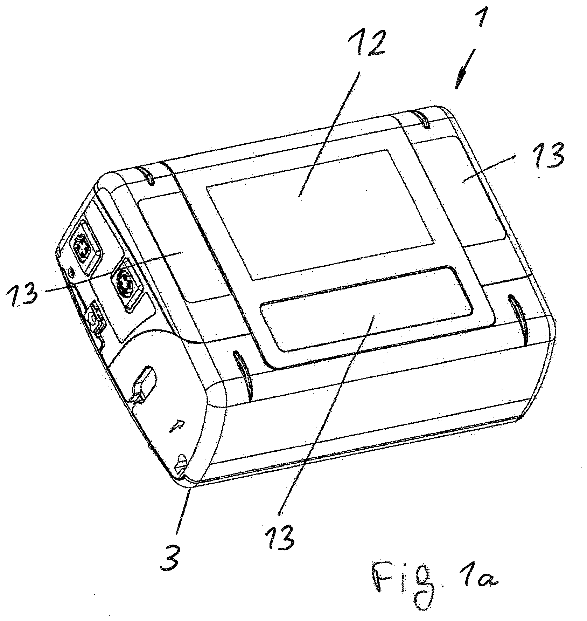

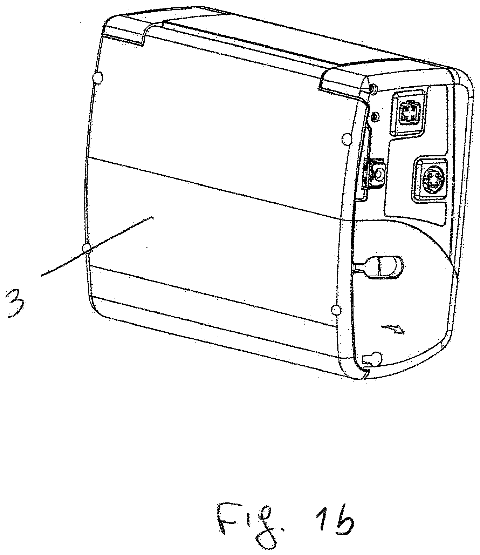

The pump 1 according to a preferred embodiment as shown in the Figs. 1 to 3 comprises a housing 2 which includes a removable and exchangeable clear plastic drug bag compartment 4 with a lid 3 lockable by a key mechanism 7, and is embodied in several sizes depending on the therapy used so as to keep the size minimal for each therapy. The drug bag compartment 4 comprises a bottom 4a which is surrounded by sidewalls 4b.

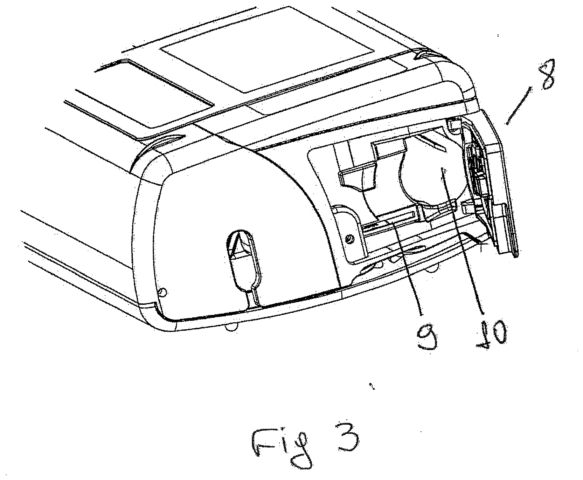

As to be seen from Fig. 3, the housing 2 of the pump 1 further comprises a battery compartment 10 with a disposable or rechargeable main battery and an internal rechargeable secondary battery (not shown). Two alkaline batteries in series are used as standard, but alternatively e.g. a rechargeable LiPo battery pack with separate connecting pins can be used (not shown), so that it is not possible to recharge alkaline batteries and, hence, to avoid errors. A battery door 8 when open allows access to a SIM card 9 for a GSM communication module (not shown).

The pump 1 additionally includes a smaller safety rechargeable battery (not shown), so that the pump 1 can even run during a battery change and alarm when the main battery has been depleted. This secondary battery is recharged by the main battery or an external power pack cable which is to be connected to the pump 1 and also charges the battery pack, while an external charger for replacement battery packs also exists.

The pump 1 includes a dual microcontroller electronic control for redundancy safety (not shown), and, as schematically depicted in Fig. 1a, further comprises an LCD display 12 and keys 13 with backlight, wherein the dual microcontroller electronic control is further adapted to control said display 12 and said keys 13 and optionally further components not shown.

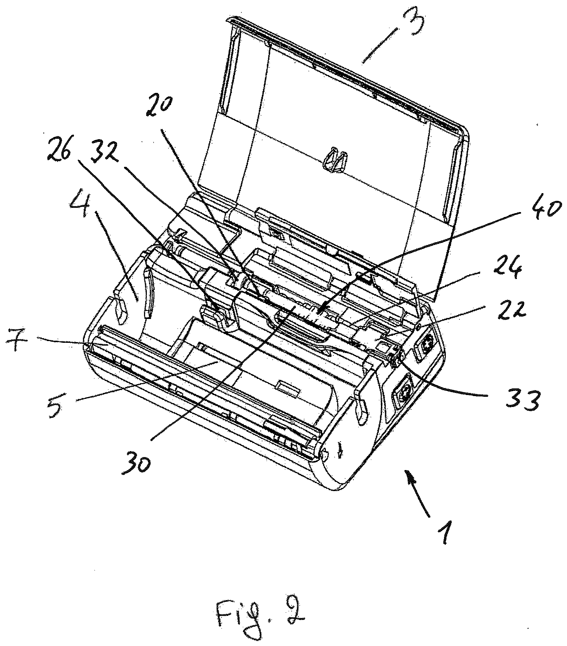

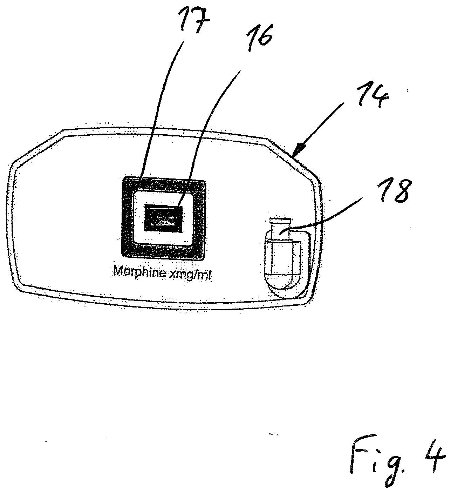

As also shown in Fig. 2, the pump 1 further comprises an RFID reader 5 provided at the bottom 4a of the bag compartment 4. Additionally or alternatively, the reader 5 can be provided at at least one of the sidewalls 4b of the bag compartment 4. So, any tagged bag 14, as exemplarily shown in Fig. 4, which bag is provided with a tag or label 16 and an outlet 18 and is put into the bag compartment 4, can be read so as to make an infusion protocol selection automated and exclude medication errors. According to a system disclosed in EP 2 659 923 A1, an analgesia bag prefilled by a pharmaceutical company for this pump 1 is read automatically with the drug name and concentration guiding the pump 1 to find the correct protocol in an embedded or cloud-based drug library, by passing several steps of programming making it easy for nurses at home who are not trained on this pump 1 to perform an infusion excluding errors in preparation and programming, wherein there may be also the need of a second nurse (mandatory for safety in some countries).

According to the preferred embodiment shown in Fig. 4, the tag 16 on the pharmaceutical bag 14 is put (not with adhesive but) inside a pocket 17 formed by the same bag material, which pocket is thermally bonded on the bag material enclosing the tag 16 with its antenna. The bag 14 is printed with written contents externally as usual on the flip side as also to be seen from Fig. 4. This solution is advantageous to avoid contamination of the drug with adhesive glue material after a longer time, in particular years, of storage. The outlet 18 of the drug bag 14 preferably includes a valve (not shown) which is opened by an infusion set connector (not shown), so that the drug bag 14 sold through pharmacies needs an infusion set sold through a pump 1 distribution network so as to connect with its outlet 18, also having infusion segment and an downstream accessories like filters and extension line tubing. This is a drug-device combination for a pharmaceutical product dedicated to a specific pump.

In order to help verify infusion and alarm conditions, the pump 1 includes an upstream pressure sensor 20 and a downstream pressure sensor 22, as to be seen from Fig. 2. As further shown in Fig. 2, an infusion pump segment 30 is inserted into the pump 1. The infusion pump segment 30 is disposable and forms a fluidic path from the outlet 18 of the drug bag 14 (Fig. 4) when inserted into the drug bag compartment 4 to an outlet. The infusion pump tube segment 30 comprises an upstream inlet 32 which is adapted to be coupled to the outlet 18 of the drug bag 14 by means of a fluid connector or connecting tubing (not shown) and further comprises a downstream outlet 33 for outputting a fluid drug or medication from the drug bag 14. As further to be seen from Fig. 2, the infusion pump tube segment 30 is to be arranged so that the upstream pressure sensor 20 is provided near the inlet 32 of the infusion pump tube segment 30 and the downstream pressure sensor 22 is provided near the outlet 33 of the infusion pump tube segment 30.

As further shown in Fig. 2, the pump 1 comprises a pump mechanism module 40 which is configured as a linear peristaltic mechanism and adapted to engage a portion of the infusion pump tube segment 30 so as to generate at least one squeezed point in said tube portion and to move it in the direction towards the outlet 33 of the infusion pump tube segment 30. So, the infusion pump tube segment 30 is to be arranged within the pump 1 so as to be subject by engagement of the pump mechanism module 40.

The pump 1 comprises means to reduce false alarms for air in line; these are very often in hospitals and home settings and are very annoying. In the embodiment shown in Fig. 2, said means comprise an air in line detector or sensor 24 downstream of the pump mechanism module 40 at the infusion pump tube segment 30 so as to sense the absence of liquid causing a bag empty alarm, and an additional air in line detector or sensor 26 after an air eliminating filter (not shown) arranged inside the drug compartment 4, so as to eliminate the alarm generated by said air in line sensor 24 if no air is detected downstream and only bubbles but not full air is present at said air in line sensor 24. The additional air in line sensor 26 is provided for detecting air after the air eliminating filter in a connecting tubing (not shown) to be positioned in the drug compartment 4 and connecting the outlet 18 of the drug bag 14 to the inlet 32 of the infusion pump tube segment 30 in some models for therapies like parenteral nutrition which usually generate a lot of air bubbles caused by an emulsion. There is also a detection if a filter is defective (not shown), and in said case alarms are given for a defective filter if bubbles are detected by the additional air in line sensor 26 preferably provided as mentioned above.

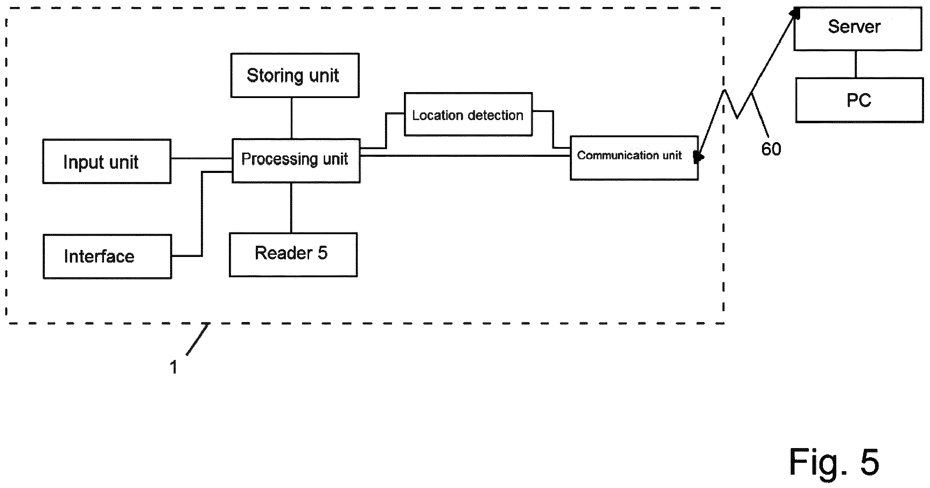

Fig. 5 shows a schematic block diagram of an internal electronic circuit of the pump 1 comprising a processing unit to which connected are the reader 5, an interface unit including the LCD display 12, an input unit including the keys 13, a storing unit and a communication unit. Further, there is provided a location detection unit which is coupled between the communication unit and the processing unit. In the shown embodiment, the communication unit is coupled with a remote server to which a PC terminal is connected, wherein said connection 60 is preferably made via the internet.

According to a preferred embodiment, the programming steps for a tagged drug are: Turning the pump on, initializing the pump, and placing the drug bag 14 into the compartment 4, wherein this usually overrides a first screen on the pump 1. In case the same drug has been infused last time with a volume being more than a near-end-of-infusion-volume in the bag, it is asked if the infusion is resumed or started as a new one. If assumed by the pump algorithms or chosen a new one by the user, a drug name concentration and protocols found for one or more therapies and profiles are displayed, and the nurse choses (by scrolling through a few found protocols or more possibly by using one only found protocol) the correct therapy/profile and protocol and validates by using a start/stop button so that the infusion starts. In case the patient himself is provided with a tag, the nurse puts his tag into the compartment 4 before placing the drug bag 14 therein, so that the therapy and profile are set through a drug library or interoperability communication with an EMR. In case of the existence of a protocol list for a known patient/profile and therapy, the protocol is found and proposed right away; it cannot be simpler for not trained freelancer nurses at home care who have to deal with many different pump makes. This is an inverse search of the drug library database as normal programming that goes "therapy > profile > drug > protocol". Knowing the drug, there are therapies which do not appear, and there are also protocols which are not relevant to the specific drug and, hence, do also not appear, so that a search in lists is easier. Since the RFID label 16 can also be self-adhesively put at any drug bag 14 and even a custom number for the compound can be written in the drug library, any drug can be delivered so easily according to the preferred embodiment of the present invention so that many problems on home care drug infusions are solved. So, a simple tag reader innovation leads to an invention of a new tag reader aided programming mode, that simplifies a pump use being otherwise complex. There is a lot of writing about artificial intelligence, and simple forms of it may be used for this programming sequence as well as algorithms that are looking like artificial intelligence from outside but they are not.

RFID tag printers exist on the market and are able to print a self-adhesive label with letters. While programming a tag behind the print, there are difficulties to connect them with a remote server (like MicrelCare server) or hospital drug/time schedulers (like CERNER/EPIC) in order to print the label 16 as unique custom No. Since the same drug library is used by all those systems, only one unique or individual custom No. can be associated with "therapy/profile/protocol", so that after reading the pump 1 has only one choice to infuse resulting in a central signification of the nurse's job. Alternatively, a home care provider can remove the "therapies/profiles/protocols" files being not relevant to his job or individual patient he treats (e.g. the palliative care has no child profile) from a hospital drug library it is sharing, so that the effect of user simplicity is the same, since the nurse has only one option to choose and infuse.

According to a preferred embodiment, the pump 1 is connected via a WiFi and GSM communication with an external cloud-based server to transmit infusion data, therapy feedback data and alarms, and to receive drug library and protocol list data through secure encrypted communication, wherein location tracking for a lost pump or asset management in hospitals is performed. The communication is subject to TCP/IP and SSL/TLS safety on top. GSM and WiFi work separately or together as known from EP 2 881 875 A1 to find an external or internal location of the pump 1 in a hospital. Location tracking associated with drug RFID recognition and infusion data complete a compliance file to be sent back to the patient's electronic health record (EHR) after infusion is finished successfully. The frequency of communication is lower with battery operation and higher with mains operation. The patient home WiFi UN & password can be programmed on the pump 1, but also sent through the web application wireless or by cable at a connected place (hospital/home care provider) including several available pumps and automatically used by the pump 1 to ease use by untrained users at home. If WiFi is not available, a GSM network is used. The connected server the pump 1 is communicating with is interoperable so that the pump 1 can receive a prescription protocol and/or a drug library database or protocol list from different places in a hospital, wherein its alarms can go to hospital alarm system, and send patient compliance data back to EHR after infusion is completed or report to the insurance company etc.. This way with connectivity at server site entrains the pump software to be better managed, and changes and/or responses to customer demands are done faster. The server can be installed in a hospital, but more effectively in the cloud where all other hospital data are soon or later going to be.

Pump communication:
A preferred embodiment is about a connected infusion pump 1 through several communication channels. Communication can consume much of the available battery energy, so that according to a preferred embodiment several communication power consumption reduction methods are used.

First, according to a preferred embodiment, the RFID performs a reading operation only at the start-up process and is then kept silent or performs reading at long intermittent periods to reduce power. The pump 1 communicates though only one channel at a time to reduce power.

The WiFi and GSM systems consume more energy at opening a communication with the server, wherein actually the spending of power is more than the message to be sent which in the present case is tiny. So, when setting the preferences of the pump configuration, the user can adjust the intermittent time which is mandatory for such a communication, in particular 5 or 15 or 30 minutes. This is the mandatory time where the pump 1 sends anyway a status message, such as volume infused, run/stop status, and other history data.

Additionally, the pump 1 opens the communication and sends data at every event such as start/stop of infusion, a bolus demand, a program change, or pre-alarm or alarm. This is done because these messages are rare and altogether they do not consume much and are very important to monitor therapy remotely and may need an immediate reaction from a caregiver.

Occlusion alarm is a special case which sometimes may happen repetitively, so that the present invention keeps the communication open for a while after one occlusion alarm and if no subsequent alarm occurs, it closes the communication to save battery life, whereas otherwise it sends more alarms and infusion start messages with the same communication established.

Pump programming:
According to a preferred embodiment, the pump 1 has several delivery modes defined by medical practice and bibliography: Continuous, Volume/time, Continuous + Bolus, Bolus only, Auto-bolus, Ramps, 25 steps.

At start, the pump 1 needs to know if the last infusion needs to resume, if a priming set is needed, if the patient is the same or has changed, and if the program is new or the same. If a "bag change" is chosen the last used protocol appears. For entering a new program in a pump it is needed to enter a safety code that allows permission to change parameters.

The pump 1 can be programmed by four ways (in contrast thereto from the prior art it was known only manual and drug library programming) as allowed by a user permission code, in increasing error prevention order:
- Manual programming, wherein an infusion mode and each parameter are edited by a user without any limit; this is the historical way of pump programming and the default if no drug library or protocol list has been downloaded.
- Progamming the drug library, wherein a user selects the therapy, then the profile, then the drug and then the protocol which includes a delivery mode and default parameters, and can edit protocol parameters within specified limits, wherein, however, this does not modify the drug library.
- Programming a protocol short list in order to limit and simplify a drug library to just what a specific patient will use, wherein the user can edit protocol which is stored for future use.
- Programming an RFID tag drug, wherein the drug and its concentration cannot be changed from what is read, and the user most usually just selects the protocol proposed since this is the safest infusion programming, but, if the code permission allows, editing of other parameters, except concentration, can be done. If a tagged bag 14 is entered into the pump drug container compartment 4, it automatically supersedes all other programming ways. The tag 16 may also contain a protocol with limits if the user does not want to download a drug library.

RFID reader use:
According to a preferred embodiment, the pump 1 also comprises an RFID reader 5 arranged close to the drug bag compartment 4, that reads an RFID tag 16 embedded in or adhesively put to the bag 14 and automatically selects from the drug library the correct protocol for the user that can also be scanned by the RFID reader 5 by placing the patient's tag close to the drug compartment 4. The system according to the preferred embodiment is intended to be used for drug bags prefilled by pharmaceutical companies and sold through the so-called pharmaceutical route, wherein the RFID tag 16 is embedded on or in the drug bag surface, or for drug bags 14 including a compounded drug produced in a compounder facility or hospital pharmacy, wherein said drug bags are provided with a self-adhesive label 16 including written information combined with RFID data. In a database it is created a No. to be read from the RFID tag 16 that corresponds to a custom (compounded) drug and is unique for the compounder and the specific drug/infusion protocol. Whereas in several countries, two nurses are needed to make sure that the correct drug and protocol are delivered to a patient, according to the present invention, the RFID reader 5 provides the verification ID back to the server so that the system allows a single nurse for the treatment using telemedicine as there is a double check. Errors in morphine preparation onsite resulting from both dilution errors and programming an erroneous concentration are frequent and sometimes fatal. The present invention prevents an erroneous drug delivery.

Drug library:
A drug library is a list of drugs per concentration and their soft and hard infusion limits for a therapy specialized for a specific patient or delivery route or other therapy specificity or variant called therapy profile. Therefore, limits for a child and an adult are different for the same drug and therapy. Technically it may have the form of a database that can be queried by several ways. The drug library is difficult to be implemented in a pump 1 with limited keys 13, so that it is set up on a PC and transferred to the pump 1 by cable or wirelessly. The PC is a terminal of a cloud-based monitoring and programming application (e.g. with commercial name "MicrelCare"). There are different drug libraries (depending on limits and drugs used) per hospital care area or home care facility, each having preferences on safety limits and drugs used.

Historically in prior art, drug libraries have been set up to reduce medication errors by limiting infusion parameters like maximum infusion rate per therapy, drug, and profile, for stationary large volumetric pumps for hospital use. These are adapted to a hospital environment and not to an outpatient setting. According to the present invention, this drug library has been modified for better usability and safety in home care as needed for an ambulatory pump and its usual therapies while also adapting the RFID automatic programming from a given drug library.

According to a preferred embodiment, while the standard infusion protocol search path "therapy + profile (subcategory of therapy with specificities for a patient or practice) + drug" is used, features have been added which are especially useful for home care setting.

A therapy feedback with relevant questions is programmed per therapy; indeed, at home care, the nurse cannot ask the patient how he feels as it is practice in the hospital.

Also, the user can select some protocols from the drug library and put them into a most used protocol list as a separate menu. In this way, a chronic patient who takes a parenteral nutrition at home does not need each time to carry out a full library search, but select a hydration or "yellow" or "white" bag (arbitrary protocol names for water solutions, aminoacids, lipids solutions) drug protocols for his next infusion. Since the drug library is for hospital use each change does not overwrite the protocol in the drug library, in contrast thereto according to the preferred embodiment each change overwrites the protocol, so that each time, when a patient or user modifies a protocol from a protocol list, this appears next time he uses it.

According to a preferred embodiment, the drug libraries include a pump configuration, i.e. general pump settings, for infusion not at protocol level, but at therapy level, easing home care use that usually is for one therapy only.

Description of the drug library creation on a server:
In the web application, MENU > Clinical settings: There is under it a selection line with

### THERAPIES - DRUGS - DRUG LIBRARY - PROTOCOL LISTS - QUESTIONNAIRES - PUMP FILES

Select: as underlined

### THERAPIES - DRUGS - DRUG LIBRARY - PROTOCOL LISTS - QUESTIONNAIRES - PUMP FILES

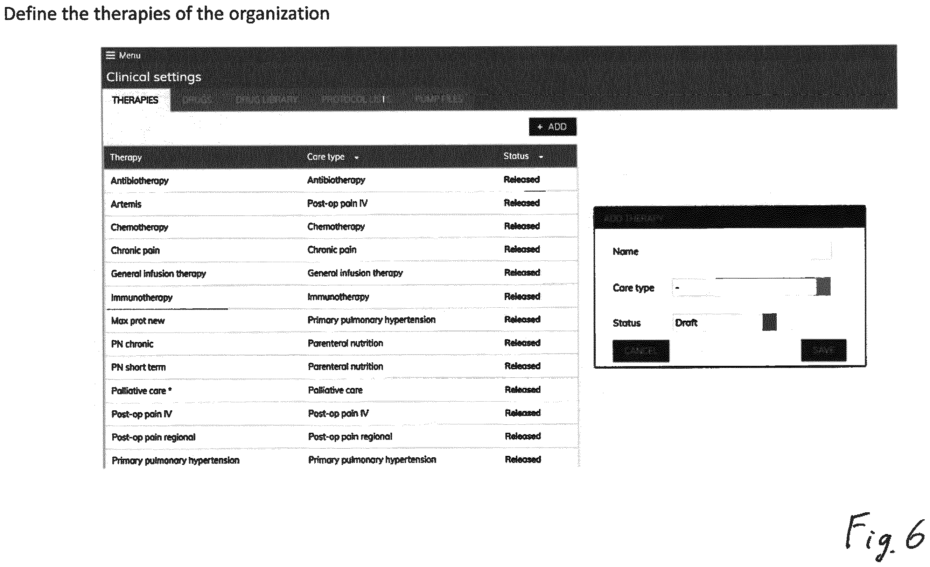

Under THERAPIES (Fig. 6) there is a window to add a therapy and a list of
Therapy - Care Type - Status
The Therapy ist described by a subjective name as given by the user for said therapy.

The care type is a pre-defined therapy name under which the representation of a specific infusion and therapy data is shown on a server monitoring therapy page, that is not only for one infusion, but for a sequence or history of infusions showing the evolution of the therapeutic effects in time. Such data are: Infusion data, alarm data with time stamp, volume per drug/nutrition type data, answers to questions per time stamp data, and downstream pressure data with time stamp.

The Status describes if it is released or not. In prior art pumps infusion events like infusion history data as volume delivered and protocol used as well as an alarm history are internally stored. A connected pump 1 according to the present invention stores those events also on a distant server, so that events may stack up to a long history of many consequent infusions during long periods of time, together with answers to questions, as therapy feedback. Therapy review monitoring screens are pre-defined as Care Type on CLINICAL SETTINGS > THERAPIES so that all information per Care Type are meaningful to the therapist.

### THERAPIES - DRUGS - DRUG LIBRARY - PROTOCOL LISTS - QUESTIONNAIRES - PUMP FILES

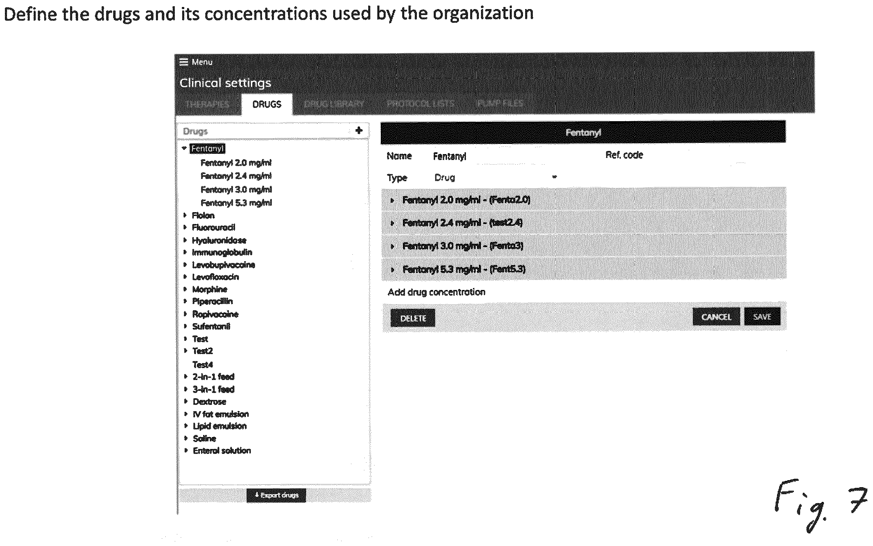

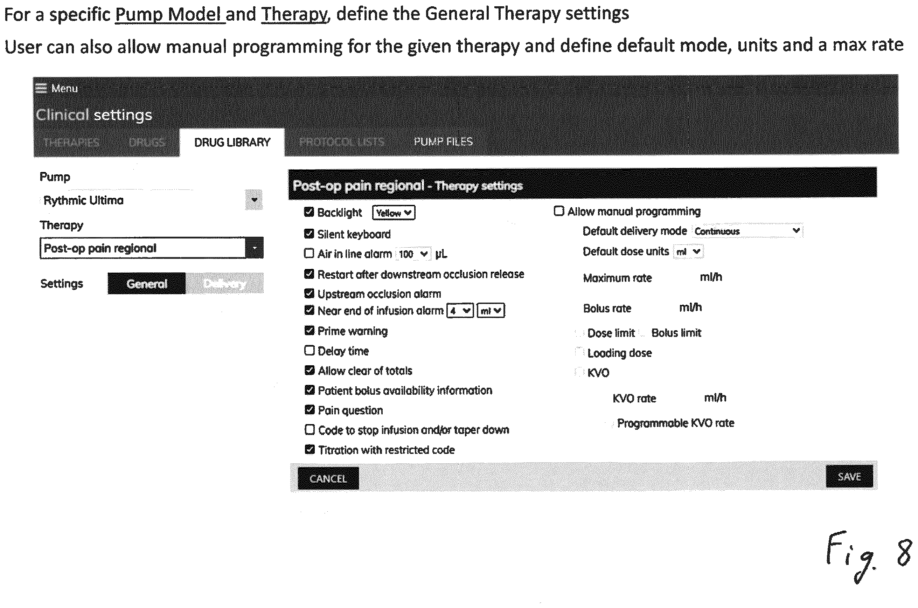

Under DRUGS (Fig. 7) there is a long list of all used by care area drugs, and under GENERAL (Fig. 8) the infusion configuration parameters are edited, and for each drug a window shows Name and Ref. Code so that an RFID or Bar-Code No. can be associated to a drug name. In the window below all concentration variations of the drug are shown, wherein the user can add more, each with its "Ref. Code". "Ref. Code" can consist of a letter before a number to show that an RFID tag 16 is mandatory, and the pump 1 recognizes that and does not allow drugs without any RFID tag to be infused. This is useful to the need of two nurses to verify the protocol which in this case are not needed any more, or in case of untrained nurses and level of trust a doctor may have on home care treatments.

### THERAPIES - DRUGS - DRUG LIBRARY - PROTOCOL LISTS - QUESTIONNAIRES - PUMP FILES

Under Drug Library on the left choose a pump model and a therapy are chosen.

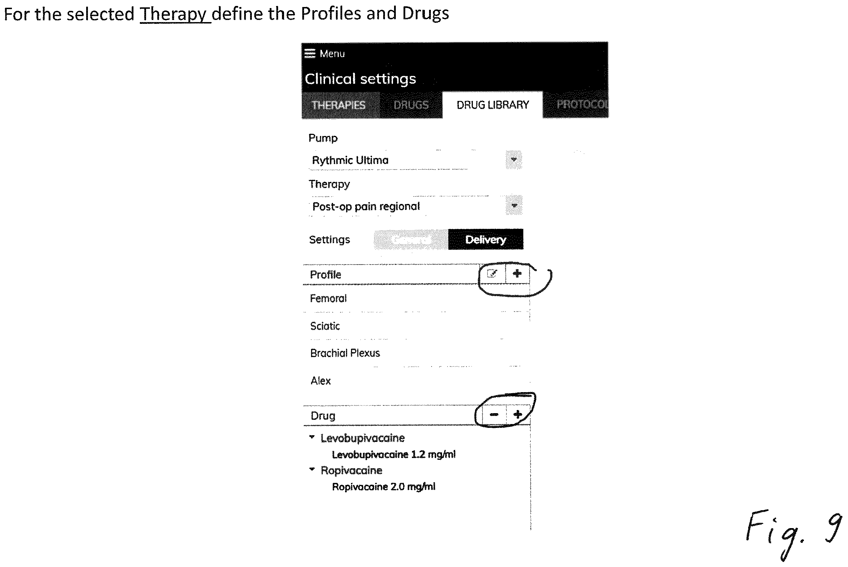

Under Settings (Fig. 9) a Profile of therapy as a sub-set of Therapy, and then a Drug from a short list can be added.

Under selection GENERAL, all pump general infusion settings, i.e. the general pump behavior (configuration), and alarms are given.

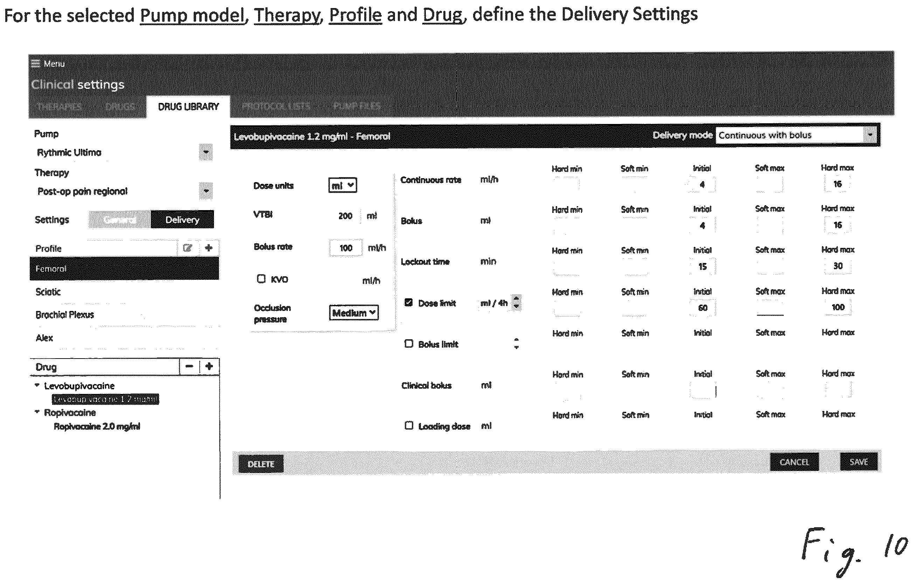

Under DELIVERY (Fig. 10) once a profile and a drug have been selected, a protocol page appears, with a pop-up menu for Delivery Mode to infuse, and once selected then all protocol parameters and units are to be edited in a screen adapted to the selected delivery mode.

### THERAPIES - DRUGS - DRUG LIBRARY - PROTOCOL LISTS - QUESTIONNAIRES - PUMP FILES

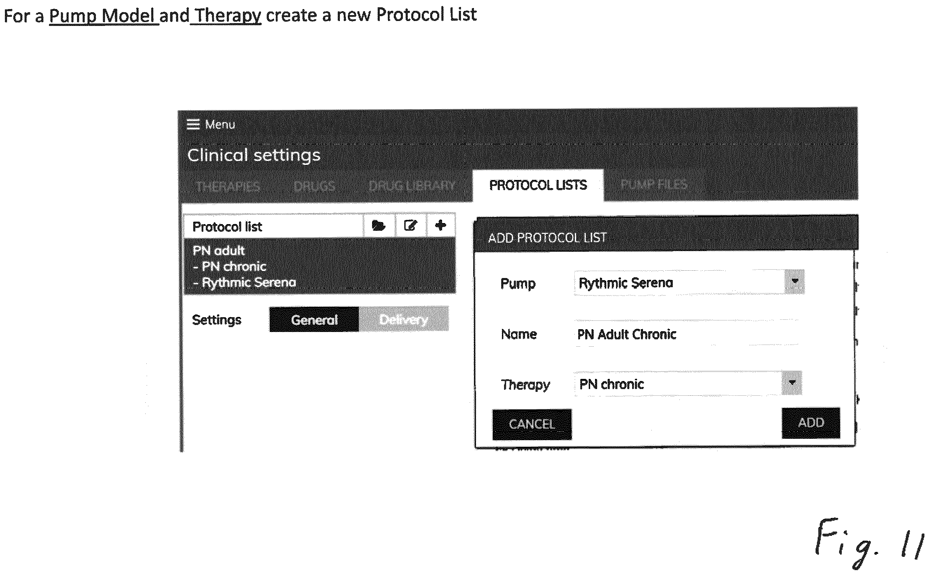

Under PROTOCOL LISTS, a full list of protocols is given, and under SETTINGS (Fig. 11) a new protocol list for a Pump model and Therapy can be added and named accordingly.

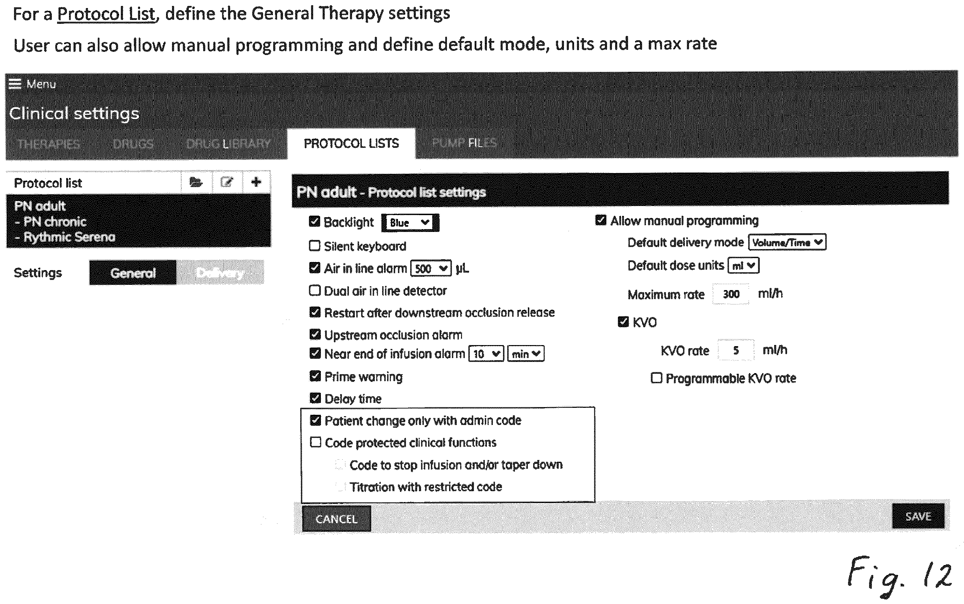

Under GENERAL (Fig. 12) the configuration of infusion, i.e. the general infusion settings being the same as with the Therapy above, is shown.

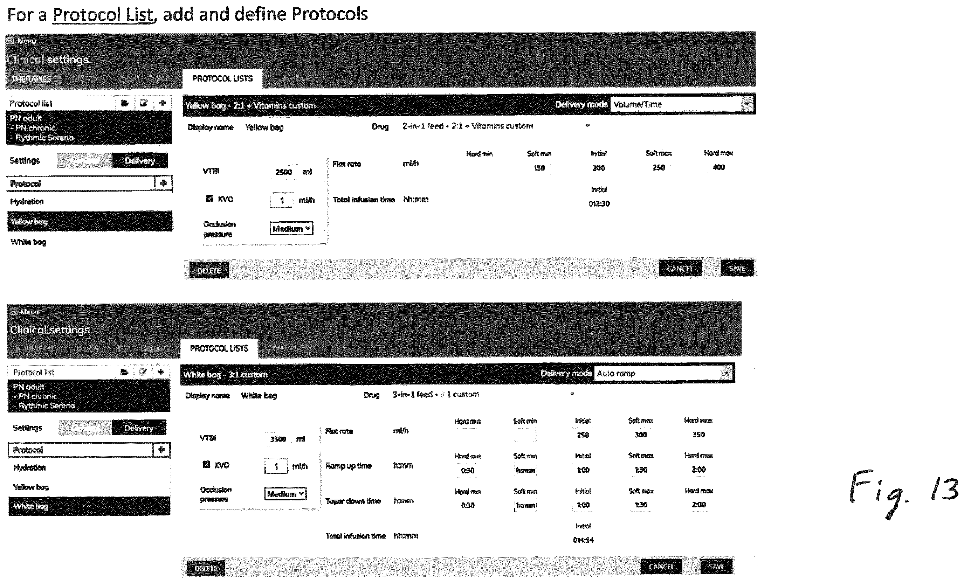

Under DELIVERY (Fig. 13) the Delivery Mode is chosen, and then adequate parameters appear to edit with limits.

So, a protocol list can be independent of the classic query "Therapy > Profile > Drug > Protocol" sequence and does not use any drug wherein a drug usually is on the name of the protocol. Compared to a drug library that requires three steps for finding a correct infusion protocol (therapy, profile and drug), a protocol list has therapy and profile on the protocol list name, and drug on the protocol name, so that a user having downloaded a list for his therapy and profile just has to check a drug from the protocol list to infuse, wherein less steps in the user interface are needed to be performed in order to find the correct protocol for the infusion resulting in an easing use for chronic patients who have to deal with a few protocols only and wherein the therapy and profile do not change which is suitable to the use of the pump 1 as a mono-therapy pump for a dedicated user. This is a simpler way useful in home care where chronic patients must only deal with a few protocols every day repeatedly. So, the pump 1 where a few protocols of this list are downloaded has usually max 3 protocols to be shown as in parenteral nutrition. According to the present invention, the ambulatory pump is diversified from a bedside LVP pump and classic drug library.

### THERAPIES - DRUGS - DRUG LIBRARY - PROTOCOL LISTS - QUESTIONNAIRES - PUMP FILES

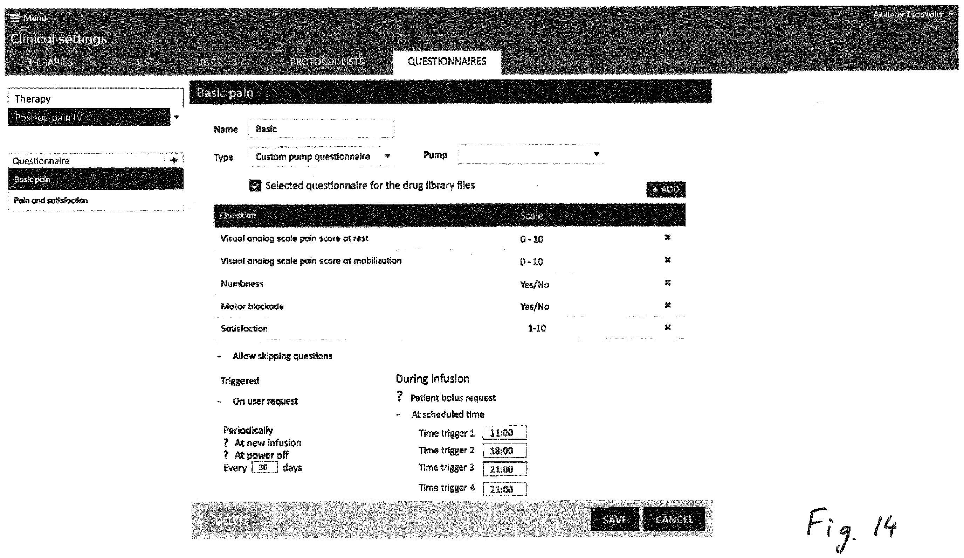

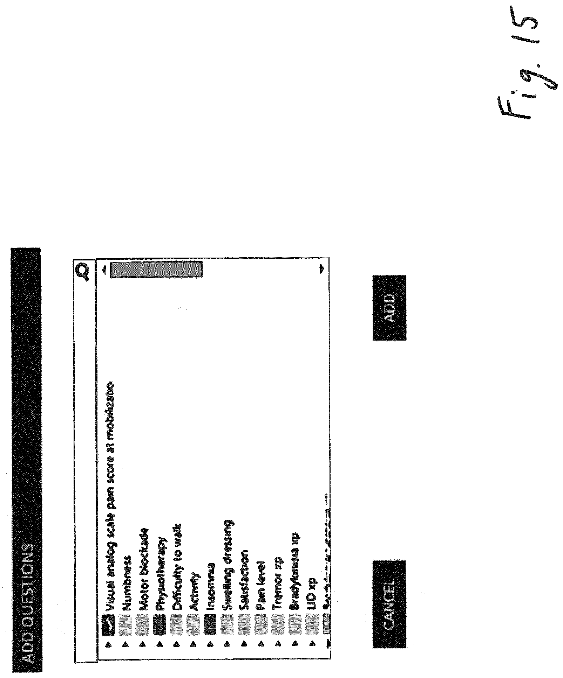

Under QUESTIONNAIRES (Fig. 14) a Therapy is chosen from a list and it is edited which questions are kept and which questions are rejected with selecting a feedback scale for each. Questions from a drop-down menu of them can be added (Fig. 15). The selected questionnaire can be attached to a patient name (so his therapy is known) on MicrelCare, and downloaded with drug library, or attached to the protocol list which can also be sent through internet.

According to a preferred drug library setting, standard questions for the user as therapy feedback are provided (as e.g. described in EP 2 410 448 A1 and EP 3 217 304 A1) which the doctor choses for a specific therapy profile, that can be more standard or may be a therapy combination treatment and needs customization. The frequency and time of the appearance of the questions on the pump screen is a definable parameter in the drug library. Since the pump 1 is interconnected as an internet of things (I.o.T) device, the answers together with infusion data and alarms are written in a database on the server and presented per infusion or series of consequent infusions, so that the doctor can better visualize the therapy progress since the feedback is presented on a graph, too. In the case of e.g. parenteral nutrition, the doctor can see what nutrition types have been delivered last month on a graph, with downstream pressure and catheter near occlusion alarm algorithm results (pressure data processing to predict imminent occlusion), or nausea, or glucose level side effects in case of erroneous ramp type infusion setting. A new protocol can be sent via the internet, and the pump 1 reads it and sends it back to the server so that the doctor is assured that the correct protocol has arrived and been stored in the pump 1, and then the doctor sends an acknowledgement so that the user can use it safely. This way a total therapy feedback and treatment correction is made remotely.

In each protocol, if it is in the drug library setup, a questionary will preferably appear at time defined in the drug library. The patient answers the questions on the screen with two options: "Yes/No" or by means of a scale to be defined (usually 1 to 10). The type of the scale is defined in the drug library setup. The answers are sent back to the server as described elsewhere.

### THERAPIES - DRUGS - DRUG LIBRARY - PROTOCOL LISTS - QUESTIONNAIRES - PUMP FILES

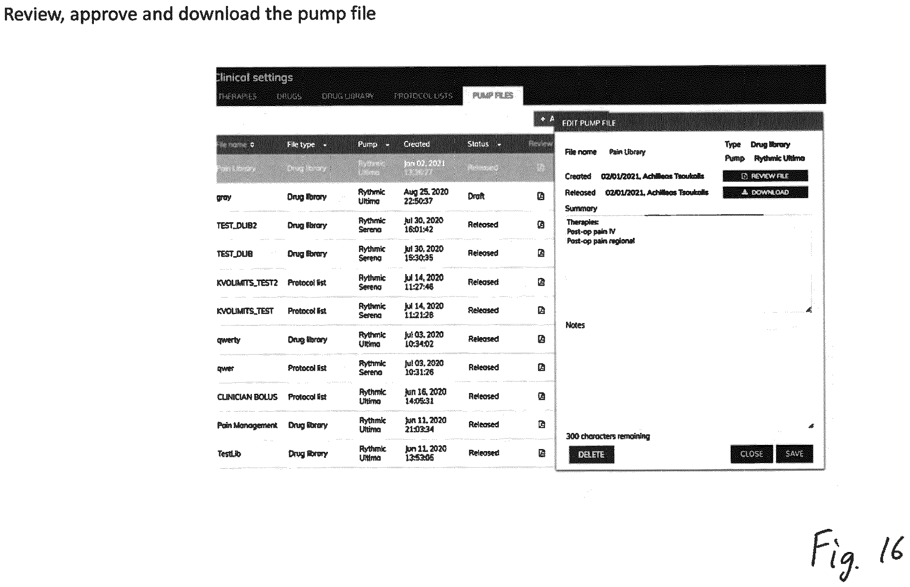

This is a file of part of a big drug library to be downloaded on a pump for a specific patient or care entity. As shown in Fig. 16, the file includes FILE NAME, FILE TYPE drug library or protocol list, PUMP MODEL, DATE CREATED, STATUS if it is a draft or released, REVIEW what revision is, DOWNLOADED if yes or no by the pump 1.

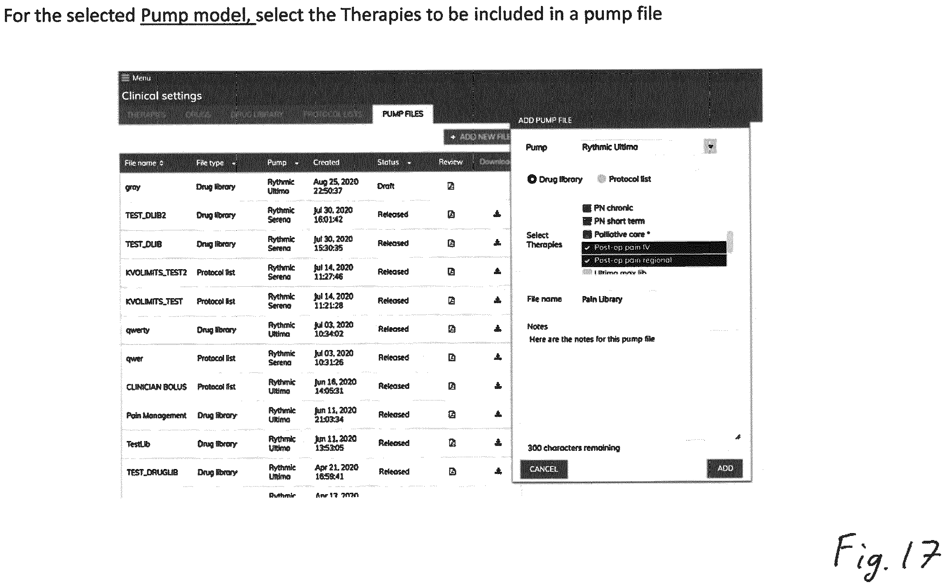

Under PUMP FILES, a list of pump files is shown. "Add a file" opens fields: Pump model, Check points, Drug Library or Protocol List, Pop-Up menu of therapies to choose to be included in the file, and File Name and Notes. Checking a file opens an editing file window as shown in Fig. 17 where it is to be entered a date created and released, all selections above for the pump 1 and therapies are to be done, and buttons to REVIEW and UPLOAD are to be clicked.

Pressing UPLOAD, an Uploader window is opened as shown in Fig. 20 with a summary of the file, and uploading can begin and verified.

So, the pump 1 receives only therapies relevant to the unit they serve.

Description of the use of drug library uploaded at the pump 1:
The user interface of the pump 1 is adaptable to the type of the downloaded database. So, if a new program is selected at the start menu, if no database requires a manual programming, and if a drug library is present, a protocol selection through the drug library follows; but in a first step, manual programming can be done if allowed in configuration-safety settings of the pump 1.

Drug library programming:
The pump 1 shows a list of available therapies wherein the user selects one.

Then a list of profiles is shown wherein the user selects one.

Then a list of drugs is shown wherein the user selects one.

Then the available protocols are shown wherein the user selects an appropriate one. He can adjust settings within soft and hard limits, but adjustment is not saved for a next programming (not next infusion after a change of the bag 14 where it remains same).

Protocol list programming:
The pump 1 displays a short list of available protocols.

These protocols usually indicate two or three drugs a patient needs to get during a therapy, like in case of parenteral nutrition a) hydration, b) amino acids called "yellow bag" and c) lipids called "white bag". Each drug and/or nutrition type delivered is recorded in the server and showed in a time scale for the doctor's review.

This preferred approach greatly facilitates the use of an ambulatory pump for chronic patients with one or a few drugs to be administered in sequence.

Due to this type of programming and because of the nature of those treatments, any change in a protocol is noted in the protocol list and is therefore proposed next time. This is because therapies at home care are evolving and not static. The protocol follows the evolution of the therapy that can be long or the whole lifetime wherein even the age is changing.

Manual programming:
The pump 1 asks for the delivery mode, and thereafter the next programming steps depend on the delivery mode selected. Then the user edits the concentration and volume of the drug.

So, the programming in a continuous mode is much shorter than in a ramps mode, wherein step up, continuous and then descent to zero ml/h have all to be edited.

RFID programming:
The processing unit of the pump 1, once a tagged bag 14 has been put into its drug compartment 4, checks if this drug with its concentration is the same as the last one delivered. If so, then it is asked if there has been a change of the bag 14 so that in such case the same protocol will be used again, and, if not, it is gone directly to the new program.

Case: Protocol list downloaded:
The protocol list as described according to a preferred embodiment (compared to a drug library) combined with RFID scanning reduces significantly the programming steps for a nurse. So, it allows a single nurse (instead of two in some European countries) to program the pump 1. After scanning, if there is no bag change, the pump 1 displays the drug and concentration and most probably, since the doctor selects only the relevant protocols to be uploaded to the pump 1, only one therapy and protocol will appear for the nurse to select resulting in rendering the programming as easiest as possible. She then can directly approve the protocol proposed and start infusion with minimum steps, so that no training is needed. If more protocols are shown, even then the list is significantly reduced by eliminating all those for different drugs and associated delivery modes. For example, it is very unlikely that morphine administered mostly in case of palliative care and terminal cancer is used for a child profile. For a scanned drug, editing of the protocol (within limits) is not permanent, and default values reappear with the next programming. As described above, this is not the case for not scanned drugs used for chronic patients. So, in accordance with the preferred embodiment, a home care provider having a bank of pumps 1 dedicated to those two therapies may upload only a few protocols, so that medication errors are greatly reduced and the training of the nurses as process is extremely simple.

Case: Drug library downloaded:
After scanning if there is no bag change, the pump 1 shows therapies and profiles available to be selected for the drug found. The list of therapies and protocols is very much reduced by knowing the drug, so that the process is easier even in this case. This is not the recommended use of the device, but it is available.

In this mode, a change of concentration of the drug is not available for safety reasons. This is for hospital use of the device. An editing of proposed infusion settings is available within limits set in the drug library.

Personalized tag:
It is possible that the pump 1 does not include any drug library or protocol list and all needed part of the drug library as limits are contained in the drug tag 16 together with the protocol. In this case, the nurse may only choose the patient profile. It is well known that a lot of data can be stored in an RFID. This is advantageous for compounded drugs produced in the hospital or by the home care provider, where the bag 14 is prepared for a specific patient, since the therapy and profile are known. A personalized tag may have a number or letter/symbol indication so that the pump 1 goes directly to the personalized tag mode. The pump 1 is adaptable to read the protocol and limits from a drug library, protocol list or tag itself. For drugs prefilled in tagged drug bags by pharmaceutical companies, personalization with reprogramming is possible, wherein only editing limits can be changed, but not the drug name and concentration.

5G NR virtual pump:
All the above are compatible with actual 3G/4G and WiFi network security that does not allow safe remote pump programming, not because of safety that is assured by SSL security, but network availability and response time especially in a hospital environment with emergencies when a nurse may monitor and remotely program many pumps from her desk. It is known that 5G networks are going to be built inside factories for automation and inside hospitals for monitoring and telematic operations like robotic surgeries, wherein according to a preferred embodiment the pump programming and remote drug library and protocols are updated in real-time.

The pump 1 of the preferred embodiment may accommodate a 5G communication module that allows a so-called virtual pump implementation, i.e., the pump 1 can be manipulated remotely. An app on a 5G mobile device can mirror the pump function, and a doctor can remotely program the pump 1 by means of the mobile device and view any historical data in order to understand problems and solve them remotely or send a nurse on site. To do this, also the cloud server (e.g. "MicrelCare") may have a 5G communication interface, so as to bypass the normal internet infrastructure that is prone to hacking and latency delays, and communicate with those pumps 1 having a 5G module through a safer 5G network. So, said app on a mobile device can be an (e.g. "MicrelCare") interface that may have this virtual pump interface being as safe as an independent app. In the same way, the remote server communicates to such an app on a mobile device that is connected through 5G only with this network, wherein risks are reduced and the response time is increased. An authentication in mobile devices by means of fingerprints and face recognition solves a big problem to allow a remote pump programming just by a doctor/nurse code. To make it clear, the server therapy monitoring service is somewhere in the cloud, distributed in one or several computers, and all preferably have direct access to the wireless 5G network and to the standard internet via fiber. Depending on the pump or app connected, communication through the same connection network, standard internet or 5G, is chosen so as to assure a fast response. Internally the server gives priority to the communication that needs a fast response such as remote programming. This priority acts like ASIO drivers do for music playing latency reduction.

5G NR (New Radio) is a radio access technology developed by the 3GPP group for the 5G (fifth generation) mobile network, wherein it has been designed to be the global standard for the air interface of 5G networks. 5G NR networks achieve a communication with ultrareliable low latency (uRLLC) for internet of things (loT) that allows a remote communication like with a direct cable connection. These networks also have a much better and further improving authorization, authentication and encryption technology that reduces hacking success to levels acceptable by regulatory bodies.

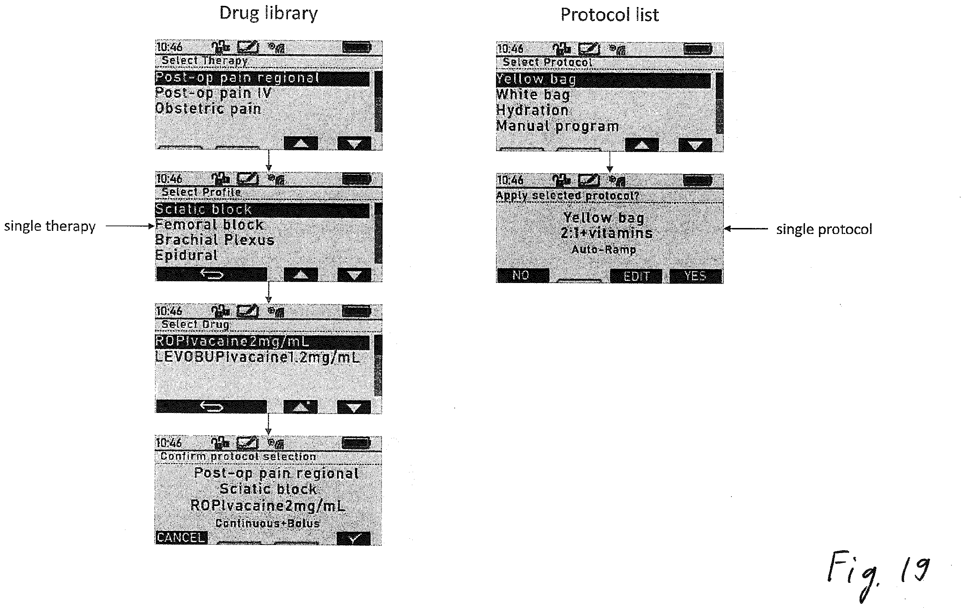

The description of each therapy or protocol are uploaded wirelessly or by cable to the pump 1 and stored in its storing unit. For programing the pump 1, its processing unit causes to display a drug library and a protocol list. An example of such a display is shown in Fig. 19 with the drug library shown at the left side and the protocol list shown at the right side. In the drug library, first the highlighted therapy is selected, then a profile (sciatic block) below is selected, then a drug (Ropivacaine) is selected, and then the protocol is displayed with all the programming steps if opened, i.e. delivery mode (continuous Infusion Rate, Continuous Volume - Time, continuous + bolus, Auto bolus, ramps, 25 steps etc.), and all parameters are needed for each selected delivery mode (this is usually called programming the pump 1). For each parameter, the drug library as downloaded indicates the selected limits in order to protect the nurse from errors. In the protocol list as shown at the right side of Fig. 19, there are one or more titles to be selected. Once a protocol title is selected, next the protocol is displayed and can be edited (EDIT) or watched and to be started (YES). Any edit done on the protocols shown are stored so that next time the last edition will be shown. This is very useful for chronic patients in particular in case of parenteral nutrition wherein a patient uses himself his own pump 1 every day, and it is useful to reduce repetitive editing.

## Claims

1. An infusion pump device, comprising a pump unit (30, 40) having an inlet (32) and an outlet (33), a connector being in fluid communication with the inlet (32) of said pump unit (30, 40), a medication reservoir accommodation portion (4) for accommodating a medication reservosir (14) having a label or tag (16) so that an outlet (18) of the medication reservoir (14) is to be coupled to said connector, and a reader (5) which is provided at said medication reservoir accommodation portion (4) so that the reader (5) is adapted to read a label or tag (16) of the medication reservoir (14) when arranged at said medication reservoir accommodation portion (4).

2. The infusion pump device according to claim 1, further comprising a housing (2) which includes at least said pump unit (30, 40), said medication reservoir accommodation portion (4) and said reader (5).

3. The infusion pump device according to claim 1 or 2, wherein said reader (5) is further adapted to read the tag or label of a patient as long as a medication reservoir (14) is absent from said medication reservoir accommodation portion (4).

4. The infusion pump device according to at least any one of the preceding claims, wherein said medication reservoir accommodation portion (4) comprises a compartment adapted to accommodate a medication reservoir (14).

5. The infusion pump device according to claim 4, wherein said compartment (4) comprises a bottom (4a) and sidewalls (4b), and wherein said reader (5) is provided at said bottom (4a) and/or at at least one of said sidewalls (4b).

6. The infusion pump device according to at least any one of the preceding claims, wherein said reader (5) is an RFID/NFC and/or barcode reader.

7. The infusion pump device according to at least any one of the preceding claims, further comprising a communication unit, in particular a wireless communication unit (like a WiFi and/or a GSM/GPS/3G/4G/5G etc. cellular wireless communication unit), adapted to communicate with a remote server.

8. The infusion pump device according to claim 7, further comprising a storing unit adapted to store at least a drug library downloaded from the remote server and including a list of different drugs, and an infusion protocol list downloaded from the remote server and including different specific infusion protocols each of which requires at least one specific drug taken from said drug library and defines a specific drug delivery and/or application to a patient.

9. The infusion pump device according to claim 8, further comprising an input unit adapted to enable a selection of a predetermined specific infusion protocol from the infusion protocol list.

10. The infusion pump device according to claim 9, wherein said input unit is further adapted to enable a modification of the predetermined specific infusion protocol, said storing unit is further adapted on the basis of said modification to amend said predetermined specific infusion protocol to a modified specific infusion protocol in the infusion protocol list stored, and said input unit is further adapted to then enable a selection of a predetermined specific infusion protocol being said modified specific infusion protocol from the infusion protocol list.

11. The infusion pump device according to any one of the claims 8 to 10, further comprising a processing unit adapted to check if the read drug data read by said reader (5) and indicating the characteristics of the drug included in the medication reservoir (14) matches with the requirements of the predetermined specific infusion protocol and only in case there is a match allows said pump unit (30, 40) to be started.

12. The infusion pump device according to claim 11, further comprising a user interface, wherein said processing unit is adapted on the basis of the read drug data to find only those specific infusion protocols which are relevant for the read drug and to present them by said user interface.

13. The infusion pump device according to claim 12, wherein said processing unit is further adapted to find from said relevant specific infusion protocols the specific infusion protocol which has been most used within a given time interval in the past and to present this specific infusion protocol at a first place in an order by said user interface.

14. The infusion pump device according to any one of the claims 11 to 13, wherein said processing unit is further adapted to select only those specific infusion protocols which in particular require the administration of a drug with the same name and concentration as with the read drug.

15. The infusion pump device according to any one of the claims 8 to 14, wherein said storing unit is further adapted to store a patient list downloaded from the remote server, and said input unit is further adapted to enable a selection of a specific patient profile from the patient list.

16. The infusion pump device according to claim 15, wherein said processing unit is further adapted to provide for said selected specific patient profile a patient-medication link for downloading a relative infusion protocol and a 5R list including patient verification, drug, infusion protocol, time of the start of the infusion and delivery route from the remote server or for reading them from the label or tag (16) of the medication reservoir (14), and to validate the specific patient and drug.

17. The infusion pump device according to claim 15 or 16, wherein said processing unit is further adapted, after having read the drug data by said reader (5), to find a predetermined specific infusion protocol from the infusion protocol list by using the specific patient profile, and in case said patient profile indicates a chronic patient to omit this step if the predetermined specific infusion protocol has already used once and the patient is the same.

18. The infusion pump device according to any one of the claims 11 to 17, wherein said processing unit is further adapted to validate the read drug by using a 5R list including patent verification, drug, infusion protocol, time of the start of the infusion and delivery route downloaded from the remote server.

19. The infusion pump device according to any one of the claims 11 to 18, wherein said processing unit is further adapted to detect from the read drug data the content of the drug as a characteristic and preferably in addition patient specific and/or infusion specific data and/or a link for downloading a 5R list including patent verification, drug, infusion protocol, time of the start of the infusion and delivery route from the remote server.

20. The infusion pump device according to any one of the claims 11 to 19, wherein said processing unit is further adapted on the basis of a selection of the therapy and the drug as well as a profile defining a subcategory of the selected therapy to provide a selection of predetermined specific infusion protocols to be used.

21. The infusion pump device according to at least any one of the claims 7 to 20, further comprising a location detection unit adapted to receive and process the signals from said communication unit and on the basis of said signals to determine the actual location of the infusion pump device.

22. The infusion pump device according to claim 21, wherein said location detection unit is further adapted to store the determined actual location.

23. The infusion pump device according to claim 22, further comprising a display, wherein said location detection unit is further adapted to still operate even when said display is deactivated.

24. A medication reservoir for the infusion pump device according to any one of the aforementioned claims, comprising a label or tag (16) and including a drug pre-filled in a pharmaceutical company or compounded in a hospital which drug in particular comprises an analgesic medication.
